# EUROPÄISCHE PATENTANMELDUNG

(11) **EP 1 433 491 A2**
(43) Veröffentlichungstag der Anmeldung: **30.06.2004**
(21) Anmeldenummer: 03029129.8
(22) Anmeldetag: 18.12.2003
(51) Int. Cl.: A61M 1/36

(54) **Vorrichtung zur Kanülierung eines Blutgefässes**

(30) Priorität: 23.12.2002 DE 10261575
(71) Anmelder: Novalung GmbH, 72379 Hechingen (DE)
(72) Erfinder: Matheis, Georg, 72393 Burladingen (DE); Frerichs, Heiko, 72379 Hechingen (DE); Sandmann, Axel, 79254 Oberried (DE); Klietsch, Dietmar, 71083 Herrenberg (DE)
(74) Vertreter: Otten, Hajo, Dr.-Ing.

(57) **Zusammenfassung**

Die vorliegende Erfindung betrifft eine Vorrichtung (20; 40; 60) zur Kanülierung eines Blut führenden Gefäßes (30; 46; 61) mit einer Kanüle (22; 42; 62), welche nach Einbringung in das Gefäß (30; 46; 61) in Fluidverbindung mit dem Gefäß (30; 48; 61) steht. Ferner ist zumindest ein Mittel (24; 44; 54; 68) vorgesehen, durch welches eine kontrollierte Aufteilung des Blutes in einen ersten, das Gefäß (30; 46; 61) durch die Kanüle (22; 42; 62) verlassenden Teilstrom und in einen zweiten Teilstrom, der in dem Gefäß (30; 46; 61) weiterfließt, erfolgt.

## Beschreibung

Die vorliegende Erfindung betrifft eine Vorrichtung zur Kanülierung eines Blut führenden Gefäßes mit einer Kanüle welche nach Einbringung in das Gefäß in Fluidverbindung mit dem Gefäß steht, sowie deren Verwendung und ein Verfahren zur Kanülierung von Blut führenden Gefäßen.

Derartige Vorrichtungen sind im medizinischen Fachgebiet hinreichend bekannt und finden bei vielen verschiedenen medizinischen Indikationen ihren Einsatz.

So werden sie bspw. in extrakorporalen Blutkreisläufen eingesetzt, wie bspw. bei der extrakorporalen Lungenunterstützung. Diese ist dann notwendig, wenn die Funktion der Lunge eines Patienten gestört ist und die Gefahr besteht, dass die Sauerstoffversorgung des Körpers beeinträchtigt ist. Bereits seit längerem werden hier Geräte eingesetzt, die - ähnlich einer Herz-Lungen-Maschine bei einer Operation am offenen Herzen - die Funktion der erkrankten Lunge zeitweise übernehmen können.

Bei der temporären extrakorporalen Lungenunterstützung (ECLA) ist die Membranoxygenation (ECMO) seit vielen Jahren ein etabliertes Behandlungsverfahren bei Patienten mit akutem schwerem Lungenversagen. Analog der Herz-Lungen-Maschine wird dabei Blut über eine weitlumige Kanüle bspw. aus einer der großen Körper-Hohlvenen entnommen, extrakorporal mittels einer Pumpe durch Membranlungen getrieben und dann über eine zweite, bspw. in der großen Körperschlagader platzierte, Kanüle zurückgeführt.

Bei Patienten mit guten hämodynamischen Verhältnissen kann sogar ein arterio-venöser Shunt geschaffen werden, der ein Lung-Assist-Device mit niedrigem Widerstand zum Einsatz ohne Pumpe erlaubt. Dieses interventionale Lung-Assist (ILA) kann ohne Einsatz von Maschinen über ein als Einweg-Produkt ausgebildetes Lung-Assist-Device den Gasaustausch der Lunge extrakorporal unterstützen (siehe bspw. Reng et al., "Pumpless extracorporeal lung assist and adult respiratory distress syndrome", 2000, The Lancet 356: 219 - 220).

Im Stand der Technik werden zur Kanülierung der Gefäße Kanülen eingesetzt, deren Größe, bzw. Durchmesser insbesondere mit Bezug auf die Gefäßgröße der Arteria femoralis ausgewählt wird.

Die Kanülengröße wird derart gewählt, dass die untere Extremität an der Kanüle vorbei weiterhin mit Blut versorgt wird.

Hierbei kann es aber, bedingt durch den traumatischen Gesamtzustand, der bspw. durch hohe Medikamentendosen, Bluttransfusionen, septische Reaktionen usw. ausgelöst wird, zu starken Kontraktionen speziell der Arterien kommen. Durch diese Kontraktionen wird der Durchmesser der Gefäße enger, wodurch sich die Arteria femoralis eng um die arterielle Kanüle schließt. Dadurch fließt das gesamte in der Arterie geführte Blut in die Kanüle und über die Kanüle in die Vena femoralis des anderen Beines, wodurch eine Blutversorgung der distalen Bereiche des arteriell-kanülierten Beines nicht mehr gesichert ist. Als Folge dieser Unterversorgung mit arteriellem, sauerstoffreichem Blut, kommt es zu einem Absterben des Gewebes unterhalb der Kanülierungsstelle, sofern die Minderdurchblutung nicht rechtzeitig erkannt und behandelt wird.

Im Stand der Technik ist verschiedenartig versucht worden, diese Komplikationen zu vermeiden. So beschrieben kürzlich Kasirajan et al., "Technique to prevent limb ischemia during peripheral cannulation for extracorporeal membrane oxygenation", Perfusion 17: 427 - 428 (2002), ein Verfahren, bei welchem zur Vermeidung von Ischämien eine arterielle Reperfusion mit einer gleichzeitigen distalen venösen Drainage angewandt wird. Dazu werden in die Arteria femoralis und die Vena femoralis jeweils zwei Kanülen mit unterschiedlicher Größe eingebracht und die arteriellen und die venösen Kanülen jeweils Y-förmig miteinander verbunden.

Ferner beschreibt Salm in seiner Veröffentlichung "Prevention of Lower Extremity Ischemia during Cardiopulmonary Bypass via Femoral Cannulation", Ann. Thorac. Surg. 63:251-252 (1997), eine Technik zur Vermeidung von Ischämien in den unteren Extremitäten, wobei hier eine Perfusion der femoralen Arterie über einen Stent erreicht wird, der mit seinem Ende seitlich an die Arterie angenäht wird.

Diese Techniken haben jedoch den Nachteil, dass die Gefäße durch die Einführung der zwei weiteren Kanülen zusätzlich verletzt und dadurch gereizt werden. Darüber hinaus ist das Verfahren durch den Einsatz von vier Kanülen sehr aufwendig.

Im Stand der Technik sind ferner zur allgemeinen Kanülierung von Gefäßen Ballonkatheter bekannt, mit denen Gefäße verschlossen werden können.

Wimmer-Greinecker et al., "Complications of Port-Access Cardiac Surgery", J. Card. Surg. 14: 240 - 245 (1999), beschreiben die Kanülierung der femoralen Arterie und Vene im Zusammenhang mit einem Herz-Lungen-Bypass, wobei hier über die arterielle Kanüle eine Ballon-Aortenklemme eingeführt wird. Für Operationen am offenen Herzen wird nach Inflation des Ballons (und dadurch durch Abklemmen der Aorta) das gesamte Blut über den Bypass geführt.

Im Stand der Technik sind darüber hinaus zahlreiche Ballon-Katheter bekannt, mit welchen es möglich ist, Gefäße gezielt zu verschließen. Dadurch ist es bspw. bei zahlreichen cardiovaskulären Operationen möglich, durch Ausblasen des Ballons, der sich dadurch an dem zu okkludierenden Gefäß anlegt, dieses komplett zu verschließen.

Derartige Ballonkatheter sind jedoch bspw. bei einer extrakorporalen Lungenunterstützung nicht geeignet, da sie die Arterie vollkommen verschließen, wodurch der Blutfluss in die unteren Extremitäten bzw. in die Bereiche unterhalb der Kanülierungsstelle gestoppt wird, was, wie oben erwähnt, zu einer Ischämie und dadurch zu einem Absterben von ischämischen Gewebe führen kann.

Aufgabe der vorliegenden Erfindung ist es daher, eine Vorrichtung der eingangs genannten Art zu schaffen, mit welcher Blutgefäße kanüliert werden können, und welche auch bei einer extrakorporalen Lungenunterstützung eingesetzt werden kann, ohne dass die Gefahr einer Ischämie von unterhalb der Kanülierungsstelle liegenden Bereichen auftritt.

Erfindungsgemäß wird diese Aufgabe dadurch gelöst, dass bei der eingangs erwähnten Vorrichtung zumindest ein Mittel vorgesehen ist, durch welches eine kontrollierte Aufteilung des Blutes in einen ersten, das Gefäß durch die Kanüle verlassenden Teilstrom und in einen zweiten Teilstrom, der in dem Gefäß weitergeführt wird, erfolgt.

Die der Erfindung zugrundeliegende Aufgabe wird auf diese Weise vollkommen gelöst.

Mit der vorliegenden Erfindung wird es ermöglicht, dass Blut, welches in einem Gefäß geführt wird, nicht insgesamt über eine Kanüle dem Körper entnommen wird, sondern vielmehr nur ein bestimmter Anteil des in dem Gefäß fließenden Blutes. Über erfindungsgemäß vorgesehene Mittel wird gleichzeitig ein anderer Teil des Blutes kontrolliert weiter in dem Gefäß geführt. Dadurch wird die Blut- und Sauerstoffversorgung von unterhalb der Kanülierungsstelle gelegenen Bereichen gewährleistet.

So kann die erfindungsgemäße Vorrichtung daher u.a. bei einer extrakorporalen Lungenunterstützung vorteilhaft eingesetzt werden, wodurch durch das zumindest eine Mittel gewährleistet ist, dass ein kontrollierter Blut-Teilstrom dem extrakorporalen Kreislauf zugeführt wird und ein kontrollierter Blut-Teilstrom im Gefäß geführt bleibt.

Unter "Kanüle" im Sinne der vorliegenden Anmeldung wird hierbei jede Vorrichtung verstanden, die in ein Gefäß eingeführt werden kann und die ein Lumen aufweist. Die Kanüle kann unterschiedliche, in der Medizintechnik vielfältig erprobte Materialien aufweisen, wie bspw. Metalle, Polyurethan, u.a. Die Kanüle stellt vorzugsweise eine drahtverstärkte Polyurethan-Kanüle dar.

Unter "Mittel" im Sinne der vorliegenden Erfindung wird dabei jede Einrichtung an der Vorrichtung verstanden, mit welchem es möglich ist, gezielt einen Teil des Blutstromes im Gefäß weiterzuführen.

Bei einer Ausführungsform der erfindungsgemäßen Vorrichtung ist bevorzugt, wenn der in dem Gefäß verbleibende Teilstrom über das Mittel an der Kanüle vorbeiführbar ist.

Dies hat den Vorteil, dass nicht das gesamte im Gefäß geführten Blut in die Kanüle geführt und somit aus dem Gefäß geleitet wird. Vielmehr wird durch das Mittel ein Teilstrom gezielt an der Kanüle vorbei in unterhalb der Kanülierungsstelle gelegene Bereiche geführt. Dadurch können diese mit Blut und Sauerstoff versorgt werden.

Hierbei ist bei einer Ausführungsform bevorzugt, wenn das Mittel ein Ballon ist, welcher die Kanüle umlaufend umgibt, und welcher eine Oberfläche mit zumindest einer rillenförmiger Vertiefung aufweist.

Unter "Ballon" ist hierbei jeder Abschnitt am Kanülenschaft zu verstehen, der einen im Vergleich zur Kanüle erweiterten Durchmesser aufweist. Dabei kann der "Ballon" extern an der Kanüle angebracht sein, oder aber einen integralen Abschnitt der Kanüle darstellen.

Unter "rillenförmige Vertiefung" ist dabei jede Veränderung bezüglich einer gleichmäßigen Oberfläche des Ballons zu verstehen, durch welche die Oberfläche des Ballons ungleichmäßig erscheint, d.h. also bspw. gewellt, eingekerbt, etc.

Bei bekannten Ballonkathetern schließen die Oberflächen des Ballons glatt an die Oberfläche der Gefäßwand an, wodurch das Gefäß komplett verschlossen und ein Blutfluss in Bereiche unterhalb der Kanülierungsstelle verhindert wird. Bei den erfindungsgemäßen Ausführungsformen wird durch die Vertiefungen der Oberfläche vorteilhaft ermöglicht, einen Anteil des im Gefäß geführten Blutes auch in unterhalb der Kanülierungsstelle gelegene Bereiche zu führen.

Die Vertiefungen ziehen sich dabei über die gesamte Länge des Ballons hinweg. Diejenigen Bereiche der Oberfläche, die zwischen den Vertiefungen liegen, sind gegenüber den Vertiefungen erhaben und drücken gegen die Gefäßwand oder den Katheter. Durch die Vertiefungen in der Oberfläche des Ballons werden rillenartige Hohlräume gebildet, über die ein Teil des im Gefäß geführten Blutes an der Kanüle vorbei in unterhalb der Kanülierungsstelle gelegene Bereiche gelangen kann.

Bei einer bevorzugten Ausführungsform ist die Oberfläche, die zumindest eine Vertiefung aufweist, dem Gefäß und/oder der Kanüle zugewandt.

Bei denjenigen Ausführungsformen, bei welchen die Vertiefungen auf der Oberfläche des Ballons angebracht ist, die der Gefäßwand zugewandt ist, fließt ein Teil des Blutes somit zwischen Gefäßwand und Ballon in unterhalb der Kanülierungsstelle gelegene Bereiche.

Sind die Vertiefungen auf der der Kanüle zugewandten Ballonoberfläche angebracht, fließt ein Teil des im Gefäß geführten Blutes zwischen Ballon und Kanüle an der Kanülierungsstelle vorbei. Dabei ist der Ballon über die ebenfalls der Kanüle zugewandten erhabenen Bereiche auf seiner Oberfläche an der Kanüle angebracht. Die Vertiefungen können aber auch auf beiden Seiten des Ballons angebracht sein, d.h. sowohl auf der der Gefäßwand zugewandten Seite, als auch auf der der Kanüle zugewandten Seite.

Mittels jeder dieser Ausführungsformen wird erreicht, dass zwischen dem Ballon und der Gefäßwand, oder zwischen Ballon und Kanüle, oder auf beiden Seiten Durchlässe verbleiben, durch welche kontrolliert ein Teilstrom des Blutes in Bereiche unterhalb der Kanülierungsstelle weiter im Gefäß geführt werden kann.

Die Oberfläche kann dabei mindestens eine, vorzugsweise mehrere Vertiefungen haben, wobei der mit diesen Vertiefungen erzielte Durchfluss über die Anzahl und Tiefe kontrolliert werden kann. Die Form der Vertiefungen kann dabei beliebig gewählt sein, bspw. in Form einer Rille oder Kerbe. Die Vertiefungen können dabei symmetrisch oder asymmetrisch auf der Oberfläche verteilt sein.

In einer anderen bevorzugten Ausführungsform ist das Mittel zumindest ein Ballon, welcher die Kanüle abschnittsweise umgibt, so dass zumindest zwei in umfängliche Richtung weisende Enden des Ballons sich in einem umfänglichen Abstand zueinander befinden.

Hierbei ist besonders bevorzugt, wenn zwei Ballone vorgesehen sind, welche die Kanüle abschnittsweise umfassen.

Bei dieser Ausführungsform umgibt der eine oder mehrere Ballone die Kanüle nicht vollständig, sondern weist vielmehr zwei in umfängliche Richtung weisende Enden auf. Diese Enden liegen in einem Abstand voneinander, so dass im dilatierten Zustand des Ballons durch diesen Abstand ein Durchlass gebildet ist, durch welchen Blut teilweise an der Kanüle vorbei geleitet wird.

Der Abstand der Enden des einen oder der mehreren Ballone, bzw. deren die Kanüle umlaufender Abschnitt, sowie deren Anordnung auf der Kanüle kann im Hinblick auf den Kanülenumfang und auf die gewünschte Durchlassmenge an Blut variiert werden. Im dilatierten Zustand wird dadurch ein Durchgang geschaffen, durch den ein Teilstrom des Blutes hindurch und somit in Bereiche unterhalb der Kanülierungsstelle gelangen können.

Darüber hinaus kann auch die Anzahl der Ballone variiert werden, mit jeweils sich zueinander im umfänglichen Abstand befindlichen Ballonenden, wobei die dadurch gebildeten Durchgänge in regelmäßigen oder unregelmäßigen Abständen festgelegt werden können.

In einer bevorzugten Ausführungsform ist der Ballon der oben ausgeführten erfindungsgemäßen Vorrichtungen dilatierbar.

Der Ballon kann dabei mit einem Fluid dilatiert werden, das über ein zweites Lumen zugeführt wird, welches mit dem Innenraum des Ballons in Fluidverbindung steht. Dieses Lumen kann bspw. in Form eines Schlauches oder einer zweiten Kanüle außen entlang der ersten Kanüle oder aber innerhalb dieser geführt werden. Über diesen Schlauch kann dann bspw. eine Kochsalzlösung für die Dilatierung des Ballons zugeführt werden.

Die Verwendung einer Kochsalzlösung hat den Vorteil, dass, sollte bspw. bei einer Verletzung des Ballons Füllmittel aus diesem austreten, keine immunologischen Reaktionen ausgelöst werden. Bei der Verwendung von bspw. Luft oder anderen Lösungen besteht die Gefahr, dass, wenn diese Fluide aus dem Ballon in das Blut gelangen, Embolien oder immunologische Reaktionen hervorgerufen und dadurch erhebliche Schädigungen und Auswirkungen im menschlichen Körper ausgelöst werden können. Durch die physiologische Kochsalzlösung wird diese Gefahr vermieden.

Bei einer anderen Ausführungsform ist bevorzugt, wenn der Ballon nicht dilatierbar ist. In diesem Fall kann der Ballon bspw. einen Abschnitt des Kanülenschafts darstellen, der im Vergleich zur Kanüle lediglich einen erweiterten Umfang aufweist.

Der Ballon kann dabei ein Material wie bspw. Latex aufweisen, oder insgesamt aus Latex gebildet sein. Dieses Material ist in der Medizintechnik hinreichend bekannt und erprobt und ist bspw. ein dünnwandiges Latexmaterial mit 250 µm Dicke.

In einer anderen bevorzugten Ausführungsform ist der in dem Gefäß verbleibende Teilstrom zunächst in die Kanüle hinein- und anschließend über das zumindest eine Mittel aus ihr herausführbar.

Hierbei ist insbesondere bevorzugt, wenn das zumindest eine Mittel ein Auslass in der Kanüle ist.

Dies hat den Vorteil, dass - bspw. bei einer Gefäßengstellung - das gesamte in dem Gefäß geführte Blut zunächst in die Kanüle hinein geführt wird, ein Teilstrom dieses Blutes wird jedoch durch zumindest einen sich in der Kanüle befindlichen Auslass wieder hinaus geführt.

Auch bei dieser Ausführungsform ist somit stets gewährleistet, dass ein Teil des Blutes abgeführt werden kann, wobei gleichzeitig die Bereiche unterhalb der Kanülierungsstelle gezielt mit Blut und Sauerstoff versorgt werden.

Hierbei ist bevorzugt, wenn der Auslass eine Öffnung in der Kanüle ist.

Derartige Öffnungen können bspw. kreisförmig sein, und sind derart gewählt, dass eine hinreichende Menge an Blut sowohl aus dem Gefäß heraus geführt werden kann, als auch eine gewisse Menge an Blut wieder aus der Kanüle hinaus in das Gefäß geleitet wird.

Die Form und Größe der Öffnungen kann dabei je nach Einsatz variieren, in Abhängigkeit davon, wie viel Blut in die unterhalb der Kanülierungsstelle gelegenen Bereiche gelangen soll. Ferner können auch mehrere Öffnungen vorliegen, aus denen Blut wieder aus der Kanüle austreten kann. Auf diese Weise kann genau kontrolliert werden, wie viel Blut aus dem Gefäß geleitet und wie viel Blut weiter im Gefäß geführt werden soll.

Ferner ist in einer anderen Ausführungsform bevorzugt, wenn der Auslass ein röhrchenförmiger Abschnitt an der Kanüle ist, der in dem Gefäß geführt ist.

Bei dieser Ausführungsform zweigt der röhrchenförmige Abschnitt von der Kanüle astartig ab. Auch bei dieser Ausführungsform kann - bspw. bei einer Gefäßengstellung, die normalerweise einen Blutfluss in unterhalb der Kanülierungsstelle gelegene Bereiche verhindern würde - das Blut kontrolliert und gezielt in Teilströme aufgeteilt werden. Das gesamte Blut fließt zunächst in die Kanüle, wonach ein Teilstrom des Blutes anschließend aus dem Gefäß geführt und ein anderer Teilstrom über den röhrchenförmigen Abschnitt wieder in das Gefäß zurückgeleitet wird.

Bei dieser Ausführungsform, die eine Art "Kanüle in der Kanüle" darstellt, kann die Länge des röhrchenförmigen Abschnitts, sein Durchmesser und der Winkel, mit dem er von der Kanüle abzweigt je nach Einsatz verändert werden. Dabei kann auch die Anzahl der röhrchenförmigen Abschnitte variieren.

In einer anderen Ausführungsform ist bevorzugt, wenn der Auslass ein kanülenartiger Abschnitt an der Vorrichtung ist, der zumindest teilweise außerhalb des Gefäßes geführt ist.

Bei dieser Ausführungsform fließt somit der Großteil des im Gefäß geführten Blutes in die Kanüle und wird aus dem Gefäß geführt. Ein Teilstrom des Blutes kann dann über einen kanülenartigen Abschnitt, der zumindest teilweise außerhalb des Gefäßes liegt, dem kanülierten Gefäß wieder zugeführt werden.

"Kanülenartiger Abschnitt" bedeutet in diesem Zusammenhang eine Einrichtung, die ähnliche Eigenschaften wie eine Kanüle oder bspw. ein Katheter aufweist, d.h. die zumindest teilweise in ein Gefäß eingeführt werden kann und die ein Lumen aufweist. Dieser kanülenartige Abschnitt kann bspw. mit einem Ende über ein Anschlussstück mit der Kanüle verbunden sein. Das Anschlussstück liegt dabei außerhalb des Gefäßes, so dass zumindest der Teil des kanülenartigen Abschnitts, der mit dem Anschlussstück verbunden ist, außerhalb des Gefäßes vorliegt. Das andere Ende des kanülenartigen Abschnitts kann in das kanülierte Gefäß eingeführt werden, wodurch ein Teilstrom des Blutes in das Gefäß zurückgeführt werden kann.

Bei einer Weiterbildung der Ausführungsformen, bei welchen der in dem Gefäß verbleibende Teilstrom zunächst in die Kanüle hinein- und anschließend über das zumindest eine Mittel aus ihr heraus geführt wird, ist bevorzugt, wenn zusätzlich ein Ballon vorgesehen ist, welcher auf der Kanüle zwischen der Eintrittsstelle des Blutes in die Kanüle und dem Auslass angebracht ist.

Diese Ausführungsform hat zum einen den Vorteil, dass mit dem Ballon das Gefäß derart verschlossen werden kann, dass sämtliches in dem Gefäß geführte Blut in die Kanüle geleitet wird, wonach über die Mittel wiederum kontrollierte Teilströme erzeugt werden. Ferner bietet der Ballon den Vorteil, dass die Vorrichtung bezüglich ihrer Lage im Gefäß festgelegt werden kann, wodurch ein Verrutschen der Vorrichtung vermieden wird.

Der Ballon kann dabei wiederum - bspw. mittels eines Fluids, welches über ein mit dem Ballon in Kontakt stehendes Lumen zugeführt wird - dilatierbar sein, oder aber einen Abschnitt auf der Kanüle mit erweitertem Durchmesser im Vergleich zur Kanüle darstellen.

Bei einer Weiterführung der oben angeführten Ausführungsbeispiele ist bevorzugt, wenn zusätzlich Mittel zum Messen zumindest eines der Teilströme vorgesehen sind.

Im Stand der Technik ist bekannt, bspw. über Sonden die Sauerstoffsättigung in den unterhalb der Kanülierungsstellen gelegenen Bereichen zu messen. Die Messung gibt jedoch lediglich an, ob der gemessene Bereich mit frischem Blut versorgt wird. Dies kann jedoch lokal über einen Umgehungskreislauf erfolgen, so dass bei diesen Vorrichtungen nicht gemessen wird, ob im kanülierten Gefäß tatsächlich ein gewisser Blutstrom und dadurch die Sauerstoffversorgung in den gesamten unteren Bereichen gewährleistet wird.

In einer weiteren Ausführungsform ist bevorzugt, wenn zusätzlich Mittel zum Regulieren der Teilströme vorgesehen sind.

Dies hat zum Vorteil, dass ― bspw. nach Messung der Teilströme durch hierzu vorgesehene Mittel ― diese Teilströme ggf. reguliert werden können. Somit kann zum einen genau bestimmt werden, wie viel Blut aus dem Gefäß abgeführt wird, und/oder wie viel Blut im Gefäß geführt bleibt. Andererseits können dann, falls dies notwendig ist, die einzelnen Teilströme über Regulierungsmittel bspw. verstärkt oder verringert werden.

So kann bspw. bei der Ausführungsform, bei welcher ein kanülenartiger Abschnitt über ein Anschlussstück mit der Kanüle außerhalb des Gefäßes verbunden ist, und bei der die Kanüle wahlweise einen Ballon aufweist, bspw. am Anschlussstück eine Messzelle angebracht sein, die die beiden Teilströme misst. Ist der Teilstrom, der über den kanülenartigen Abschnitt zurück in das Gefäß geleitet wird, unter einem bestimmten Level, wodurch eine ausreichende Sauerstoffversorgung nicht mehr gewährleistet wäre, so wird dieser Zustand erfasst. Über Regulierungsmittel kann dann ggf. die Verteilung der Teilströme reguliert werden, so dass weniger Blut aus dem Gefäß abgeführt und dadurch mehr Blut in das Gefäß zurückgeleitet wird.

Auf ähnliche Weise kann bei den anderen Ausführungsformen die zusätzliche Messeinrichtung realisiert werden. So kann bspw. der Blutteilstromes in den unterhalb der Kanülierungsstelle gelegenen Bereichen gemessen werden. Danach kann bestimmt werden, in wie weit Bedarf besteht, die beiden Teilströme zu regulieren. Wird ein verminderter Teilstrom im kanülierten Gefäß gemessen, so kann bspw. über Regulationsmittel dieser Teilstrom verstärkt werden, bspw. durch Verminderung der Dilatation eines Ballons.

Ferner ist allgemein bevorzugt, wenn die Kanüle einen Außendurchmesser von ca. 5 bis 30 French, insbesondere von 13 bis 21 French aufweist.

Bei einem Einsatz wird der Außendurchmesser der Kanüle bspw. mit Rücksicht auf das Gefäß gewählt werden, welches kanüliert wird. Die Bestimmung der Größe der Kanüle für jeden einzelnen Patienten ist wichtig, da die Größen der Gefäße von Patient zu Patient stark variieren. Vor der Punktion der Gefäße kann deren Durchmesser bspw. sonographisch ermittelt werden. So kann die Kanülengröße bspw. für die Arteria femoralis als das kritische Gefäß optimal gewählt werden. Ferner wird der Durchmesser unter Berücksichtigung der Flussmenge, die in die Kanüle geleitet werden soll bestimmt.

So beträgt in den Femoralis-Gefäßen die maximale Einführtiefe der Kanüle ca. 140 mm und die Kanülenlänge bspw. 270 mm bis 340 mm, je nach dem, ob eine Einführhilfe ("Introducer") verwendet wird, oder nicht.

Auch bei einem Einsatz von dilatierbaren Ballonen wird deren optimaler Umfang zu berücksichtigen sein und muss je nach Einsatz festgelegt werden. So kann der Ballon im dilatierten Zustand bspw. einen Außendurchmesser von ca. 1 bis 8, insbesondere von 2 bis 5 cm aufweisen.

Auch hier muss je nach Größe des zur Kanülierung vorgesehenen Gefäßes eine unterschiedliche Ballongröße ausgewählt werden, wobei die Größe in jedem Fall ausreichen sollte, dass der Ballon im dilatierten Zustand fest mit seiner dem Gefäß zugewandten Seite an die Gefäßwand drückt, damit das gesamte Blut in die Kanüle geleitet wird.

Es ist weiterhin bevorzugt; wenn die Vorrichtung mit einem biokompatiblen Material beschichtet ist.

Ein solches Material wird gewählt, um die Blutgerinnungsgefahr und Entzündungsreaktionen, die durch Kontakt der Gefäße und des Blutes mit Fremdmaterial ausgelöst werden, zu vermindern, wenn nicht sogar um diese zu verhindern. Die Vorrichtung kann bspw. mit dem von Jostra Medizintechnik, Hirrlingen, Deutschland, vertriebenen Bioline-Coating® beschichtet werden. Diese Beschichtung ist im Stand der Technik hinreichend bekannt und in der Medizintechnik mit Erfolg erprobt.

Die Erfindung betrifft weiterhin ein Verfahren zur Kanülierung eines Blut führenden Gefäßes, wobei die erfindungsgemäße Vorrichtung verwendet wird.

Die Erfindung betrifft ferner ein Verfahren zur extrakorporalen Lungenunterstützung mit einem Lung-Assist-Device, wobei die erfindungsgemäße Vorrichtung eingesetzt wird.

Unter "Lung-Assist-Device" wird hierbei jede Vorrichtung verstanden, mit der ein Gas-Austausch möglich ist, also mit welchem bspw. Sauerstoff angereichert und CO₂ entzogen werden kann, wie bspw. ein Oxygenator.

Bei diesem Verfahren wird bspw. die Arteria femoralis mit der erfindungsgemäßen Vorrichtung kanüliert, wodurch es möglich ist, das Blut sowohl teilweise in einen Lung-Assist-Device zu leiten, als auch teilweise in die unterhalb der Kanülierungsstelle gelegenen Bereiche. Über einen Schlauch wird Blut dann teilweise zum Lung-Assist-Device, bspw. einem Oxygenator, geführt, dort mit Sauerstoff angereichert und CO₂ entzogen, und über einen weiteren Schlauch und eine weitere Kanüle bspw. der Vena femoralis zugeführt.

Die erfindungsgemäße Vorrichtung ist insbesondere auch bei der pumpenlosen, arterio-venösen Lungenunterstützung vorteilhaft. Bei diesem Verfahren kann durch den Wegfall einer Blutpumpe und aufgrund kürzester Anschlussverbindung und geringer Füllvolumina die Bluttraumatisierung reduziert werden. Ferner hat diese pumpenlose Lungenunterstützung den Vorteil, dass keine zusätzlichen Maschinen zur Aufrechterhaltung des Kreislaufes notwendig sind. Der arterielle Patientenmitteldruck bewirkt bei der Verwendung entsprechender Kanülen einen ausreichenden Durchfluss, wobei das Blut im Lung-Assist-Device mit Sauerstoff aufgesättigt und das enthaltene Kohlendioxid ausgewaschen wird. Eine wesentliche Voraussetzung hierfür ist, dass die Herztätigkeit ausreichend und lediglich die Lungenfunktion beeinträchtigt ist.

Außerdem betrifft die Erfindung die Verwendung der neuen Vorrichtung zur Kanülierung eines Blut führenden Gefäßes.

Ferner betrifft die Erfindung die Verwendung der erfindungsgemäßen Vorrichtung in einer extrakorporalen Lungenunterstützung mit einem Lung-Assist-Device.

Bei dieser Verwendung kann die Vorrichtung bspw. im Zusammenhang mit einem pumpenlosen Lung-Assist-Device in der extrakorporalen Lungenunterstützung eingesetzt werden. Ein Lung-Assist-Device in Form eines Membranoxygenators ist bspw. kommerziell erhältlich von der Anmelderin, der Nova Lung GmbH, Hechingen, Deutschland. Die Besonderheit dieses Heparin-beschichteten Lung-Assist-Devices liegt in seinem sehr geringen Widerstand bezüglich einer bestimmten Blutströmung, weshalb es gerade aufgrund dieser Tatsache möglich ist, ihn zwischen der Arteria femoralis und der Vena femoralis zu positionieren. Mit der neuen Vorrichtung kann ferner die Blutversorgung von unterhalb der Kanülierungsstelle gelegenen Bereichen sichergestellt werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung des Aufbaus einer extrakorporalen Lungenunterstützung an einem Patienten;
- Fig. 2a: eine schematische Darstellung einer Ausführungsform der erfindungsgemäßen Vorrichtung;
- Fig. 2b: einen schematischen Querschnitt durch die Ausführungsform aus Fig. 2a;
- Fig. 2c: einen schematischen Querschnitt durch eine weitere Ausführungsform;
- Fig. 3: eine schematische Darstellung einer weiteren Ausführungsform;
- Fig. 4: eine schematische Darstellung einer weiteren Ausführungsform der erfindungsgemäßen Vorrichtung;
- Fig. 5: eine schematische Darstellung einer weiteren Ausführungsform.

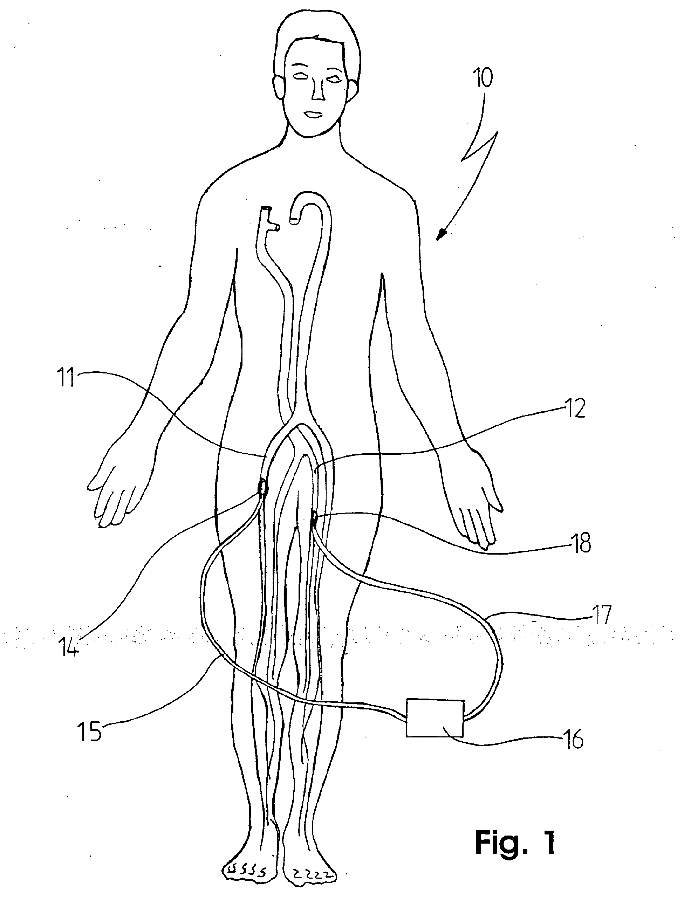

In Fig. 1 ist mit 10 ein Patient gezeigt, bei welchem mit 11 die Arteria femoralis und mit 12 die Vena femoralis bezeichnet sind. Über eine Kanüle 14 wird der Arteria femoralis 11 Blut entnommen. Ein Schlauch 15 führt von der Kanüle 14 zu einem Lung-Assist-Device 16 und ein weiterer Schlauch 17 vom Lung-Assist-Device 16 zu einer weiteren Kanüle 18 in der Vena femoralis 12.

Fig. 1 zeigt das System der extrakorporalen Lungenunterstützung bei einer femoralen Anschlusstechnik. Alternativ ist bspw. auch ein Anschluss über die Arteria femoralis 11 zur Vena jugularis möglich. Die Kanüle 14 wird mit einem der Arteria femoralis 11 des Patienten 10 optimal angepassten Durchmesser ausgewählt, und in die Arteria femoralis 11 als kritisches Gefäß eingeführt. Durch den arteriellen Patientenmitteldruck wird das Blut aus der Arteria femoralis 11 über den Schlauch 15 zum Lung-Assist-Device 16 geführt. Ein geeignetes Lung-Assist-Device in Form eines Membranoxygenators ist bspw. bei der Anmelderin, der Nova Lung GmbH, Hechingen, erhältlich. Mittels dieses Lung-Assist-Device 16 wird das Blut mit Sauerstoff angereichert und gleichzeitig Kohlendioxid ausgewaschen. Das sauerstoffreiche Blut verlässt den Lung-Assist-Device 16 über den Schlauch 17 und wird der Vena femoralis 12 über die Kanüle 18 zugeführt.

An der Eintrittsstelle der Kanüle 14 in die Arteria femoralis kann das Blut bei einem Einsatz der erfindungsgemäßen Vorrichtung teilweise an der Kanüle 14 vorbei, bzw. durch sie hindurch wieder in die Arteria femoralis 11 geführt werden, weshalb das Blut auch in das gesamte untere Bein gelangen kann. Durch diesen Kreislauf wird gewährleistet, dass bei einer schwachen oder sogar ausgeschalteten Lungenfunktion die Sauerstoffversorgung in allen Bereiche des Körpers gewährleistet ist.

Aufgrund der kurzen Leitungslänge des Systems, gemessen von Kanülenspitze zu Kanülenspitze, besteht kein Bedürfnis, einen Wärmetauscher in diese Lungenunterstützung einzuschalten. Sämtliche Komponenten können außerdem biokompatibel (bspw. mit Bioline-Coating® beschichtet) sein. Als Kanülen kommen perkutan mit Seldinger-Technik gelegte Kanülen zur Anwendung (bspw. Nova Lung BE-AVC 17-01). Die venöse Rückführung erfolgt ebenfalls über eine arterielle Kanüle, da sie aufgrund ihrer kurzen Länge einen geringeren Strömungswiderstand gegenüber einer vergleichbaren venösen Seldinger-Kanüle hat.

Die extrakorporale Lungenunterstützung kann über mehrere Tage bis Wochen hinweg angewendet werden (in Extremfällen bspw. sogar 20 bis 29 Tage lang).

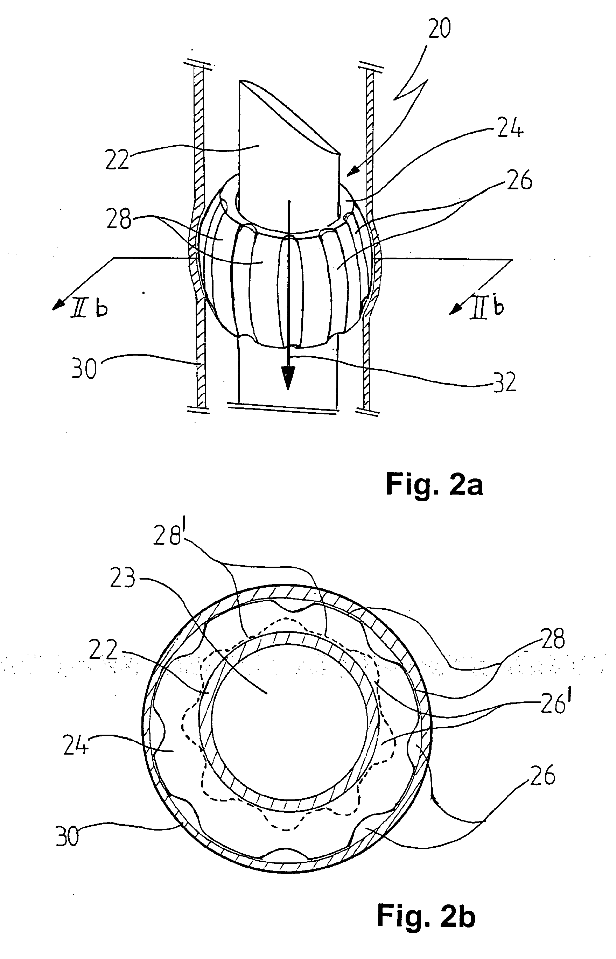

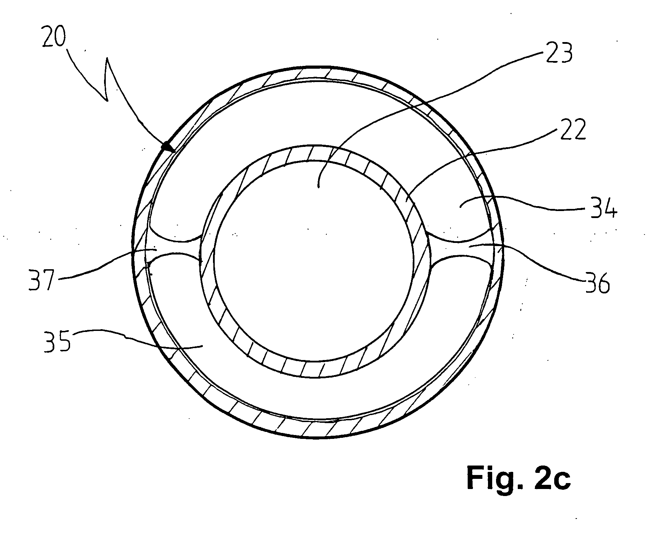

In den Figuren 2a, 2b und 2c sind Ausführungsformen der erfindungsgemäßen Vorrichtung schematisch dargestellt. Hierbei sind gleiche Elemente mit gleichen Bezugszeichen bezeichnet.

In der schematischen Darstellung in Fig. 2a und in der Querschnittsdarstellung der Fig. 2b ist mit 20 die Vorrichtung insgesamt gezeigt. Diese weist eine Kanüle 22 mit einem Lumen 23 und einem die Kanüle 22 umgebenden Ballon 24 auf. Der Ballon 24 wiederum weist Durchlässe 26 in Form von rillenförmigen Vertiefungen auf, die parallel zur Kanülenoberfläche, bzw. zum Gefäß verlaufen und sich längs über die gesamte Oberfläche des Ballons in Blutflussrichtung ziehen. Durch die Durchlässe 26 bilden sich erhabenen Stellen 28, die - wie in Fig. 2b gezeigt - direkt an die Wand eines Gefäßes 30 angrenzen.

Die Anzahl der Durchlässe 26 auf dem Ballon 24 kann je nach Bedürfnis variiert werden, ebenso wie die Tiefe oder die Form der Durchlässe.

Nach Einbringen der Kanüle 22 in ein Gefäß 30 kann der Ballon 24 mittels eines Fluids, bspw. mit einer physiologischer Kochsalzlösung, dilatiert werden. Im dilatierten Zustand des Ballons 24 drücken dessen erhobene Stellen 28 an die Wand des Gefäßes 30. Dadurch wird das Blut teilweise in das Lumen 23 der Kanüle 22 gezwungen und bildet dort einen Teilstrom, der das Gefäß 30 durch die Kanüle 22 verlässt. Teilweise kann das Blut jedoch auch über die Durchlässe 26 an der Kanüle 22 vorbei fließen, wodurch ein Teilstrom gebildet wird, der in dem Gefäß 30 weiterfließt, und welcher bspw. mit dem Pfeil 32 gezeigt ist.

Durch die Durchlässe 26 ist es somit möglich, dass ein großer Teil des Blutes in das Lumen 23 der Kanüle 22 geführt und aus der Kanüle 22 hinaus geführt wird und dass gleichzeitig das Blut teilweise über die Durchlässe 26 an der Kanüle 22 vorbei gelangt. Das im Lumen 23 geführte Blut kann dem extrakorporalen Blutkreislauf zugeführt werden. Blut, welches über die Durchlässe 26 an der Kanüle 22 vorbei fließt, kann in Bereiche unterhalb der Kanülierungsstelle gelangen und diese Bereiche mit Sauerstoff versorgen.

Die Durchlässe 26 auf der Oberfläche des Ballons 24 können in einer anderen denkbaren Ausführungsform bspw. auch auf der der Kanüle 22 zugewandten Seite als Durchlässe 26' ausgebildet sein. Der Ballon 24 wird hier über die dann ebenfalls der Kanüle 22 zugewandten erhabenen Stellen 28' an der Kanüle 22 bspw. durch Kleben angebracht. Die der Wand des Gefäßes 30 zugewandte Oberfläche des Ballons 24 ist dann glatt und schließt insgesamt an die Wand des Gefäßes 30 an.

Denkbar ist selbstverständlich auch, dass beide Oberflächen des Ballons 24, d.h. sowohl die der Wand des Gefäßes 30 als auch die der Kanüle 22 zugewandte Oberfläche, Durchlässe 26, 26' in Form von Vertiefungen aufweisen.

Statt eines Ballons mit einer "gerillten" Oberfläche kann bspw. auch ein oder mehrere Ballone vorgesehen sein, welche die Kanüle jeweils abschnittsweise umgeben und deren Enden sich in einem bestimmten radialen Abstand voneinander befinden. Der Querschnitt einer derartigen Ausführungsform ist in Fig. 2c gezeigt. In der Vorrichtung 20 sind zwei Ballone 34 und 35 auf der Kanüle 22 bracht Die Enden der Ballone 34, 35 sind in einem Abstand voneinander entfernt, so dass Durchlässe 36 und 37 gebildet werden. Über diese Durchlässe 36, 37 kann das Blut somit teilweise an der Kanüle 22 vorbeigeführt werden. Der Abstand der Enden der Ballone 34 und 35, sowie die Anordnung der Ballone 34, 35 auf der Kanüle 22 kann dabei variieren, je nach erwünschter Blutdurchflussmenge. So können die Ballone 34, 35 bspw. auch versetzt auf dem Schaft der Kanüle 22 angebracht sein.

Sind die Ballone dilatierbar, so können sie entweder über ein gemeinsames Lumen oder jeweils über getrennte Lumen dilatiert werden.

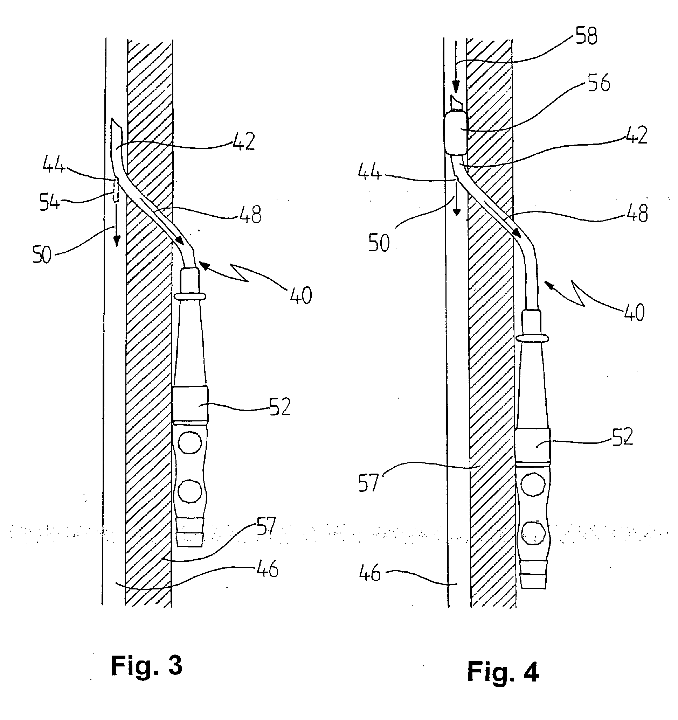

In Fig. 3 ist mit 40 insgesamt eine Ausführungsform der erfindungsgemäßen Vorrichtung gezeigt. Die Vorrichtung 40 weist eine Kanüle 42 auf, und ferner in der Kanüle 42 einen Durchlass 44 in Form einer seitlichen Auslassöffnung. Die erfindungsgemäße Vorrichtung 40 ist in einem Gefäß 46 gelegen.

Die Kanüle 42 wird mit ihrem distalen Ende in das Gefäß 46 eingeführt, wodurch der größte Teil des Blutes in die Kanüle fließt. Ein Teilstrom des Blutes, gezeigt mit dem Pfeil 48, verlässt das Gefäß 46 durch die Kanüle 42, ein Teilstrom, gezeigt mit dem Pfeil 50, fließt durch den Durchlass 44 aus der Kanüle 42 wieder in das Gefäß 46 und wird in diesem weitergeführt. Dadurch ist gewährleistet, dass - selbst wenn bei einer Kanülierung eine Gefäßengstellung im Bereich der Kanüle 42 hervorgerufen würde und der gesamte Blutstrom in die Kanüle 42 gezwungen wird - stets ein bestimmter Teilstrom 50 die Kanüle wieder verlassen und zurück ins Gefäß 46 gelangen kann. Mit 52 ist ein Anschlussstück bezeichnet, über das weitere Einrichtungen und Mittel an der Vorrichtung angebracht werden können, wie bspw. Schläuche, weitere Kanülen, Messzellen u.a.

In Fig. 3 ist ferner gestrichelt eine andere Ausführungsform gezeigt, bei welcher anstelle einer Auslassöffnung zumindest ein Durchlass durch einen röhrchenförmigen Abschnitt 54 gebildet ist. Wie bei der Ausführungsform mit der Auslassöffnung kann über diesen Abschnitt 54 ein Teil des Blutes wieder aus der Kanüle herausgeführt werden.

Eine ähnliche, jedoch erweiterte Ausführungsform ist in Fig. 4 gezeigt, wobei hier gleiche Elemente mit den gleichen Bezugszeichen wie in Fig. 3 bezeichnet werden.

Die Vorrichtung 40 weist neben der Kanüle 42 und dem Durchlass 44 zusätzlich einen Ballon 56 auf, der bspw. über ein Lumen in Form eines dünnen Schlauches, der parallel zur Kanüle 42 geführt ist, mit einem Fluid dilatiert werden kann. Das Fluid ist vorzugsweise eine physiologischen Kochsalzlösung. Der dilatierte Ballon 56 drückt mit seiner einer Gefäßwand 57 zugewandten Seite an die Gefäßwand 57 und blockiert somit den Blutfluss in die unterhalb der Kanülierungsstelle gelegenen Bereiche. Dadurch fließt sämtliches Blut in die Kanüle 42, was durch einen Pfeil 58 dargestellt ist. Der Teilstrom 48 des Blutes verlässt das Gefäß 46 über die Kanüle 42, der Teilstrom 50 tritt aus dem Durchlass 44 aus und wird im Gefäß 46 weiter geführt.

Der Ballon fungiert bei dieser Ausführungsform somit als eine Art "Abstandshalter" der Kanüle von der Gefäßwand. Über den dilatierten Ballon 56 lässt sich gleichzeitig die Kanüle 42 im Gefäß 46 festsetzen.

Auf diese Weise wird - wie in der in Fig. 2 gezeigten Ausführungsform - gewährleistet, dass der Teilstrom 48 des in dem Gefäß 46 geführten Blutes über die Kanüle 42 dem extrakorporalen Kreislauf zugeführt werden kann und der Teilstrom 50 des Blutes in das Gefäß 46 zurückgeführt wird. Mit Bezug auf Fig. 1 wird bei Verwendung der erfindungsgemäßen Vorrichtung 40 der Teil des Blutes, der über die Kanüle 42 entnommen wurde, über den Schlauch 15 dem Lung-Assist-Device 16 zugeführt, in diesem mit Sauerstoff angereichert und über den Schlauch 17 und die Kanüle 18 wieder in die Vena femoralis eingeführt. Der andere Teilstrom 50 des Blutes verlässt die Kanüle 42 über den Durchlass 44 und versorgt daher die Bereiche, die unterhalb der Kanülierungsstelle liegen mit Blut und somit mit Sauerstoff.

Der Durchlass 44 kann dabei in Form, Größe und Anzahl variieren, je nach erwünschter Blutdurchflussmenge.

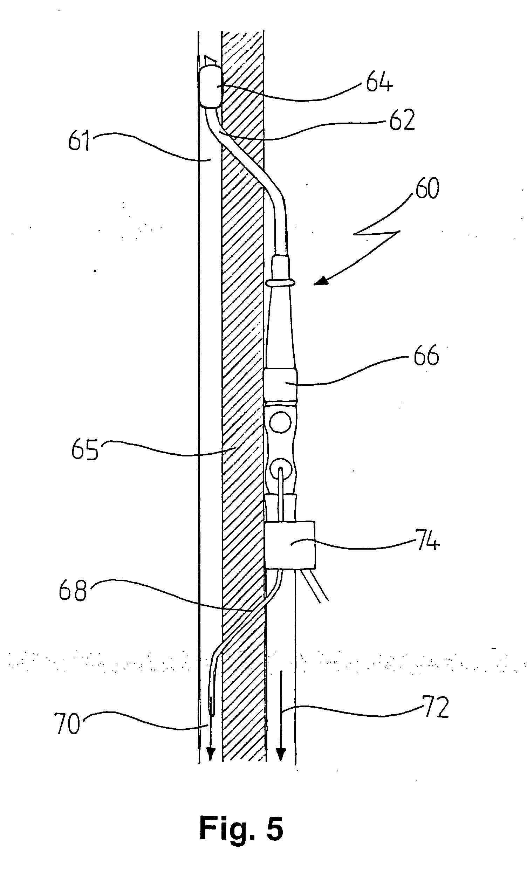

Fig. 5 zeigt eine schematische Darstellung einer weiteren Ausführungsform der erfindungsgemäßen Vorrichtung.

In der Fig. 5 ist mit 60 die Vorrichtung insgesamt gezeigt. Diese weist - in einem Gefäß 61 liegend - eine Kanüle 62 mit einem Ballon 64 auf.

Im dilatierten Zustand drückt der Ballon 64 mit seiner einer Gefäßwand 65 zugewandten Oberfläche gegen die Gefäßwand 65 und zwingt dadurch das Blut in das Lumen der Kanüle 62.

Das Blut wird über einen Abschnitt der Kanüle 62 zu einem Anschlussstück 66 geleitet, an das ferner ein kanülenartiger Abschnitt 68 angebracht ist. Über diesen kanülenartigen Abschnitt 68 kann ein Teilstrom in das Gefäß 61 zurückgeleitet werden und dadurch die unterhalb der Kanülierungsstelle gelegenen Bereiche mit Blut versorgen, was durch einen Pfeil 70 gezeigt ist. Mit dem Pfeil 72 ist ein Teilstrom gezeigt, welcher das Gefäß über die Kanüle 62 und das Anschlussstück 66 verlässt. Dabei ist vorzugsweise der Teilstrom 72, der bspw. in einer extrakorporalen Lungenuterstützung einem Lung-Assist-Device zugeführt wird, stärker als der in dem Gefäß verbleibende Teilstrom.

In der in Fig. 5 gezeigten Ausführungsform ist ferner eine Messzelle 74 am Anschlussstück 66 angebracht, mit der die Teilströme 70 und 72 gemessen werden können. Ist so z.B. der Teilstrom, der über den kanülenartigen Abschnitt im Gefäß verbleibt, nachteilig vermindert, wodurch die Sauerstoffversorgung der unterhalb der Kanülierungsstelle gelegenen Bereiche nicht mehr gewährleistet ist, so kann dies über die Messzelle 74 gemessen werden und ggf. reguliert werden. Das Anschlussstück kann bespw. Polyvinylchlorid aufweisen, oder ganz aus diesem Material bestehen.

Eine Regulation des Teilstroms in die Bereiche, die unterhalb der Kanülierungsstelle liegen, kann bspw. dadurch erfolgen, dass der Teilstrom, welcher dem Gefäß 61 über den kanülenartigen Abschnitt 68 wieder zugeführt wird, verstärkt wird, und/oder dass durch "Entlüften" des Ballons 64 nicht das gesamte Blut in die Kanüle 62 geführt wird. Ferner kann bei einem Signal der Unterversorgung der unteren Bereiche die Anordnung insgesamt entnommen werden.

In den verschiedenen Ausführungsformen wird der Ballon jeweils durch Kleben an der Kanüle angebracht. Nach Einbringen der Vorrichtung in ein Gefäß kann er unter Verwendung eines Fluids, bspw. einer physiologischen Kochsalzlösung, dilatiert werden. Das Fluid wird dabei jeweils über ein zweites Lumen zugeführt, das mit dem Ballon in Fluidverbindung steht. Dieses Lumen kann dabei entweder röhrchenförmig parallel zur Kanüle verlaufen, oder aber in dieser als zweites Lumen integriert sein.

Die Vorrichtung kann in den verschiedenen Ausführungsformen ferner insgesamt biokompatibel beschichtet sein, bspw. mit der von Jostra entwickelten und vertriebenen "bioline"®- Beschichtung.

Es versteht sich, dass noch weitere Änderungen und Modifikationen denkbar sind, ohne das Gebiet der vorliegenden Erfindung zu verlassen.

## Patentansprüche

1. Vorrichtung (20; 40; 60) zur Kanülierung eines Blut führenden Gefäßes (30; 46; 61) mit einer Kanüle (22; 42; 62), welche nach Einbringung in das Gefäß (30; 46; 61) in Fluidverbindung mit dem Gefäß (30; 46; 61) steht, **dadurch gekennzeichnet, dass** zumindest ein Mittel (24; 44; 54; 68) vorgesehen ist, durch welches eine kontrollierte Aufteilung des Blutes in einen ersten, das Gefäß (30; 46; 61) durch die Kanüle (22; 42; 62) verlassenden Teilstrom und in einen zweiten Teilstrom, der in dem Gefäß (30; 46; 61) weitergeführt wird, erfolgt.

2. Vorrichtung nach Anspruch 1, **dadurch gekennzeichnet, dass** der in dem Gefäß (30; 46; 61) verbleibende Teilstrom über das Mittel (24) an der Kanüle (22; 42; 62) vorbeiführbar ist.

3. Vorrichtung nach Anspruch 2, **dadurch gekennzeichnet, dass** das Mittel ein Ballon (24) ist, welcher die Kanüle (22; 42; 62) umlaufend umgibt und welcher eine Oberfläche mit zumindest einer rillenförmigen Vertiefung (26) aufweist.

4. Vorrichtung nach Anspruch 3, **dadurch gekennzeichnet, dass** die Oberfläche mit der zumindest einen rillenförmigen Vertiefung (26) dem Gefäß (30; 46; 61) und/oder der Kanüle (22; 42; 62) zugewandt ist.

5. Vorrichtung nach Anspruch 2, **dadurch gekennzeichnet, dass** das Mittel zumindest ein Ballon (34; 35) ist, welcher die Kanüle (22; 42; 62) abschnittsweise umgibt, derart, dass zumindest zwei in umfängliche Richtung weisende Enden des Ballons (34; 35) sich in einem umfänglichen Abstand zueinander befinden.

6. Vorrichtung nach Anspruch 5, **dadurch gekennzeichnet, dass** zwei Ballone (34; 35) vorgesehen sind, welche die Kanüle (22; 42; 62) abschnittsweise umfassen.

7. Vorrichtung nach einem der Ansprüche 3 bis 6, **dadurch gekennzeichnet, dass** der Ballon dilatierbar ist.

8. Vorrichtung nach einem der Ansprüche 3 bis 6, **dadurch gekennzeichnet, dass** der Ballon nicht dilatierbar ist.

9. Vorrichtung nach Anspruch 1, **dadurch gekennzeichnet, dass** der in dem Gefäß (30; 46; 61) verbleibende Teilstrom zunächst in die Kanüle (22; 42; 62) hinein- und anschließend über das zumindest eine Mittel (44; 54; 68) aus ihr herausführbar ist.

10. Vorrichtung nach Anspruch 9, **dadurch gekennzeichnet, dass** das Mittel ein Auslass in der Kanüle (22; 42; 62) ist.

11. Vorrichtung nach Anspruch 10, **dadurch gekennzeichnet, dass** der Auslass eine Öffnung (42) in der Kanüle (22; 42; 62) ist.

12. Vorrichtung nach Anspruch 10, **dadurch gekennzeichnet, dass** der Auslass ein röhrchenförmiger Abschnitt (54) an der Kanüle (22; 42; 62) ist, der in dem Gefäß (30; 46; 52) geführt ist.

13. Vorrichtung nach Anspruch 10, **dadurch gekennzeichnet, dass** der Auslass ein kanülenartiger Abschnitt (68) an der Vorrichtung (20; 40; 60) ist, der zumindest teilweise außerhalb des Gefäßes (61) geführt ist.

14. Vorrichtung nach einem der Ansprüche 10 bis 13, **dadurch gekennzeichnet, dass** zusätzlich ein Ballon,(56; 64) vorgesehen ist, der auf der Kanüle (22; 42; 62) zwischen der Eintrittsstelle des Blutes in die Kanüle (22; 42; 62) und dem Auslass angebracht ist.

15. Vorrichtung nach einem der Ansprüche 1 bis 14, **dadurch gekennzeichnet, dass** zusätzlich Mittel (74) zum Messen zumindest eines der Teilströme vorgesehen sind.

16. Vorrichtung nach einem der Ansprüche 1 bis 15, **dadurch gekennzeichnet, dass** zusätzlich Mittel zum Regulieren der Teilströme vorgesehen sind.

17. Vorrichtung nach einem der Ansprüche 1 bis 16, **dadurch gekennzeichnet, dass** die Kanüle (22; 42; 62) einen Außendurchmesser von ca. 5 bis 30, insbesondere von 13 bis 21 French aufweist.

18. Vorrichtung nach einem der Ansprüche 1 bis 17, **dadurch gekennzeichnet, dass** sie mit einem biokompatiblen Material beschichtet ist.

19. Verfahren zur Kanülierung eines Blut führenden Gefäßes (30; 46; 61), **dadurch gekennzeichnet, dass** eine Vorrichtung (20; 40; 60) nach einem der Ansprüche 1 bis 18 verwendet wird.

20. Verfahren zur extrakorporalen Lungenunterstützung mit einem Lung-Assist-Device, **dadurch gekennzeichnet, dass** eine Vorrichtung nach einem der Ansprüche 1 bis 18 eingesetzt wird.

21. Verwendung einer Vorrichtung nach einem der Ansprüche 1 bis 18 zur Kanülierung eines Blut führenden Gefäßes.

22. Verwendung einer Vorrichtung nach einem der Ansprüche 1 bis 18 in einer extrakorporalen Lungenunterstützung mit einem Lung-Assist-Device.
